(19) Europäisches Patentamt
European Patent Office
Office européen des brevets

(11) EP 1 062 944 B1

(12) EUROPEAN PATENT SPECIFICATION

(45) Date of publication and mention of the grant of the patent:
**03.03.2004 Bulletin 2004/10**

(51) Int Cl.[7]: **A61K 7/48**, A61K 7/06, A61K 7/32, A61K 7/02, A61K 7/40, C08G 77/50

(21) Application number: **00304947.5**

(22) Date of filing: **12.06.2000**

(54) **Cosmetic material**

Kosmetisches Mittel

Produit cosmétique

(84) Designated Contracting States:
**DE FR GB**

(30) Priority: **11.06.1999 JP 16476899**
**06.06.2000 JP 2000169265**

(43) Date of publication of application:
**27.12.2000 Bulletin 2000/52**

(73) Proprietor: **SHIN-ETSU CHEMICAL CO., LTD.**
**Chiyoda-ku Tokyo (JP)**

(72) Inventors:
- **Nakanishi, Tetsuo, Shin-Etsu Chemical Co.,Ltd.**
**Matsuidacho Usui-gun, Gunma-ken (JP)**
- **Ono, Ichiro, Shin-Etsu Chemical Co., Ltd.**
**Matsuidacho Usui-gun, Gunma-ken (JP)**

(74) Representative: **Bubb, Antony John Allen et al**
**Wilson Gunn Gee,**
**Chancery House,**
**Chancery Lane**
**London WC2A 1QU (GB)**

(56) References cited:
**EP-A- 0 545 002** **US-A- 5 557 000**

- **PATENT ABSTRACTS OF JAPAN vol. 018, no. 040 (C-1155), 21 January 1994 (1994-01-21) & JP 05 262987 A (SHIN ETSU CHEM CO LTD), 12 October 1993 (1993-10-12)**
- **PATENT ABSTRACTS OF JAPAN vol. 015, no. 068 (C-0807), 18 February 1991 (1991-02-18) & JP 02 295912 A (SHISEIDO CO LTD), 6 December 1990 (1990-12-06)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

**Description**

## FIELD OF THE INVENTION

The present invention relates to a cosmetic material which contains a specified silicone compound to acquire excellent emulsification suitability and high transparency, and besides, to ensure a feeling of refreshment for the users thereof. The present cosmetic material is usable for skincare cosmetics, hair-care cosmetics, antiperspirant cosmetics, makeup cosmetics, UV protective cosmetics and so on.

## BACKGROUND OF THE INVENTION

In general the makeup is smeared with secretion from humans, such as sweat, tear and sebum. In particular, the leading cause of the makeup spoilage consists in that the powder in a cosmetic material is wetted excessively by the sebum secreted from the skin in addition to oil ingredients compounded in the cosmetic material. Therefore, with the intention of reducing the oil ingredients remaining on the skin after the makeup is done, it has been attempted to use volatile oils, such as octamethylcyclotetrasiloxane and decamethylcyclopentasiloxane, as a part of the oil ingredients compounded in a cosmetic material.

Further, the rub and the water given to the makeup constitute external factors inhibiting the makeup effect from lasting long. For the purpose of preventing the makeup from coming off by contact with aqueous substances, such as sweat and tears, or maintaining the skin protecting effect by preventing a loss of water-soluble components and sebum in the skin, it has been carried out to heighten the water repellency by mixing a silicone oil in the cosmetic material. In recent years, the silicone oils, represented, e.g., by dimethylpolysiloxanes, have been prevailingly used as an oil ingredient for cosmetic materials because of their characteristics, including light feel, excellent water repellency and high safety.

As mentioned above, dimethylpolysiloxanes possess excellent characteristics as an oil ingredient of cosmetic materials, but it cannot be said that the dimethylpolysiloxanes were successful in producing a satisfactory improvement on the water repellency of makeup cosmetics.

As to the skin care cosmetics also, there has been a demand for oil ingredients having a light feel, water repellency, good usability and a feel of cling to the skin.

On the other hand, the silicone oils produced by introducing fluoroalkyl groups into dimethylpolysiloxanes (Japanese Tokkai Hei 2-295912, wherein the term "Tokkai" means an "unexamined published patent application") have high water repellency, compared with dimethylpolysiloxanes. By increasing the content of fluoroalkyl groups therein, the silicone oils can have sufficiently high water repellency, but they come to have a heavy feel and lose solubilities in volatile oils, such as octamethylcyclotetrasiloxane and decamethylcyclopentasiloxane; as a result, the usability thereof becomes poor. On the contrary, the fluoroalkyl content decreased in order to secure solubilities in volatile oils for the silicone oils can provide neither sufficient water repellency nor a feel of cling to the skin.

## SUMMARY OF THE INVENTION

As a result of our intensive studies done aiming at developing a cosmetic material having a light feel, sufficiently high water repellency and a makeup effect of long-duration, it has been found that the one end reactive silicone-grafted silicone compounds have extremely high water repellency, solubilities in volatile oils, such as octamethylcyclotetrasiloxane and decamethylcyclopentasiloxane, and good usability as oil ingredients of a cosmetic material, and besides, the cosmetic materials using them have a light feel, thereby achieving the present invention.

While the silicone compounds produced by the addition reaction of the foregoing silicone compounds to organohydrogenpolysiloxanes are disclosed in Japanese Tokkai Hei 7-197055 as actuation silicone oils having good low temperature characteristics, it is our finding that these silicone compounds are effective ingredients of cosmetic materials.

More specifically, when these silicone compounds are used as oils for emulsions, they can exhibit excellent ability to emulsify because of their excellent compatiblity with oils used for general cosmetic materials, such as other silicone oils, ester oils and triglyceride. And the emulsions obtained can have satisfactory storage stability, so they are very effective for cosmetic use. Further, we have found that the aforementioned silicone compounds are instrumental in cleansing sebum stains and makeup stains from cosmetics of the type which are hard to come off, and the cleansing composition comprising them has a very good touch during and after cleansing treatment.

Therefore, an object of the invention is to provide a cosmetic material not only having high compatibility with other general cosmetic materials and ensuring high stability in the emulsions comprising them, thereby enabling the preparation of cosmetics having makeup effect of long duration, but also having excellent cleansing effect on both sebum stains and makeup stains of cosmetics of the type which are hard to come off, and that, ensuring a very good

touch during and after cleansing with cosmetics comprising them.

The above-described object is attained with a cosmetic material in which is mixed a silicone compound represented by the following formula (1):

$$R^1_aR^2_bSiO_{(4-a-b)/2} \quad (1)$$

wherein $R^1$ groups, which are the same or different, each represent a hydrogen atom or an organic group selected from the class consisting of alkyl groups containing 1 to 30 carbon atoms, aryl groups, aralkyl groups, fluorinated alkyl groups and organic groups represented by the following formula (2); $R^2$ groups each represent a silicone group represented by the following formula (3); a is a number of from 1.0 to 2.5; b is a number of from 0.001 to 1.5;

$$-C_cH_{2c}-O-(C_2H_4O)_d(C_3H_6O)_eR^3 \quad (2)$$

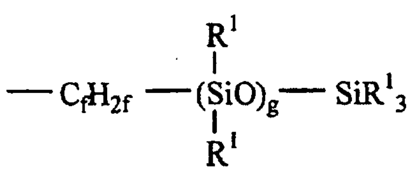

(3)

wherein $R^3$ is a hydrocarbon group containing 4 to 30 carbon atoms or an organic group represented by $R^4$-(CO)-; $R^4$ is a hydrocarbon group containing 1 to 30 carbon atoms; c is an integer of from 0 to 15, d is an integer of from 0 to 50, and e is an integer of from 0 to 50; and f is an integer of from 1 to 5, and g is an integer of from 0 to 500.

DETAILED DESCRIPTION OF THE INVENTION

The silicone compounds represented by formula $R^1_aR^2_bSiO_{(4-a-b)/2}$ (herein referred to as formula (1)) are illustrated below in detail.

Each $R^1$ group represents an organic group selected from the class consisting of 1-30C alkyl groups, aryl groups, aralkyl groups, fluorinated alkyl groups and organic groups represented by $-C_cH_{2c}-O-(C_2H_4O)_d(C_3H_6O)_eR^3$ (which is referred to as formula (2), wherein c, d and e are each an integer and fall within the following ranges: $0 \le c \le 15$, $0 \le d \le 50$, $0 \le e \le 50$; and $R^3$ represents a 4-30C hydrocarbon group or an organic group represented by $R^4$-(CO)- wherein $R^4$ represents a 1-30C hydrocarbon group).

Examples of an alkyl group as $R^1$ include a methyl group, an ethyl group, a propyl group, a butyl group, a pentyl group, a hexyl group, a heptyl group, an octyl group, a nonyl group and a decyl group.

Examples of a cycloalkyl group as $R^1$ include a cyclopentyl group and a cyclohexyl group, those of an aryl group as $R^1$ include a phenyl group and a tolyl group, those of an aralkyl group as $R^1$ include a benzyl group and a phenetyl group, and those of a fluorinated alkyl group as $R^1$ include a trifluoropropyl group and a heptadecafluorodecyl group.

Examples of an organic group represented by formula (2) include higher alcohol residues, such as residues of higher alcohols, such as cetyl alcohol, oleyl alcohol and stearyl alcohol, and polyoxyalkylene adducts thereof; higher alcohol alkenyl ether residues, and polyoxyalkylene adducts thereof; higher fatty acid residues, such as residues of oleic acid, stearic acid and behenic acid, and polyoxyalkylene adducts thereof; and higher fatty acid alkenyl ester residues and polyoxyalkylene adducts thereof.

More specifically, the formula (2) is $-O-(C_2H_4O)_d(C_3H_6O)_eR^3$ when c is 0. Further, it becomes $-O-R^3$ in the case of e=0 and d=0, and represents a residue of higher alcohol such as cetyl alcohol, oleyl alcohol or stearyl alcohol, or a residue of higher fatty acid such as oleic acid, stearic acid or behenic acid. In a case of d,e≥1, on the other hand, the formula (2) represents an alcohol residue of higher alcohol/alkylene oxide adduct (having OH at the end).

When c is 1 or 2 the formula (2) is $-C_cH_{2c}-O-(C_2H_4O)_d(C_3H_6O)_eR^3$, and this group is formed by dehydrohalogenation reaction between Si-OH group and $X(CH_2)_n-O-(C_2H_4O)_d(C_3H_6O)_eR^3$ (wherein X is halogen).

The $-C_cH_{2c}-O-(C_2H_4O)_d(C_3H_6O)_eR^3$ group in the case of c≥3 is a group introduced by the addition reaction between Si-H and a higher alcohol alkenyl ether or a fatty acid alkenyl ester (in a case of d=e=0), or a polyalkylene oxide adduct thereof (in a case of d,e≥1).

$R^2$ groups are each a silicone group represented by the following formula (3):

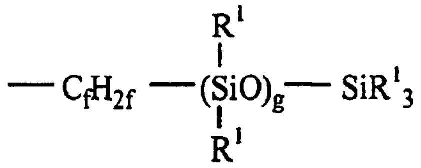

(3)

wherein f is an integer of from 1 to 5, and g is an integer of from 0 to 500, preferably from 3 to 100. For instance, f has a value of 2 when the silicone compound of formula (1) is synthesized by the reaction between a vinyl group and a SiH group. When the integer "g" in $R^2$ has a value greater than 500, such $R^2$ group is liable to have inferior reactivity in the introduction reaction into a compound constituting the main chain.

"a" in formula (1) is a number of from 1.0 to 2.5, preferably from 1.2 to 2.3, and "b" in formula (1) is a number of from 0.001 to 1.5, preferably from 0.05 to 1.0.

When the silicone compounds represented by formula (1) are used as oil ingredients of cosmetic material, the weight average molecular weight suitable therefor, though it has no particular limits, is from 500 to 200,000, preferably from 1,000 to 100,000.

The present silicone compounds represented by formula (1) can be synthesized with ease by addition reaction between organohydrogenpolysiloxanes and silicone compounds represented by the following general formula (4) and, if desired, olefin compounds as well in the presence of a platinum or rhodium catalyst, or by the reaction between silicone compounds represented by formula (5) and organovinylsiloxanes:

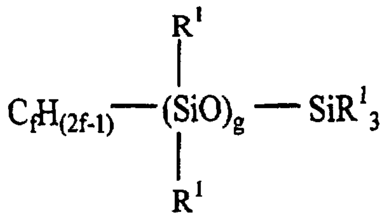

(4)

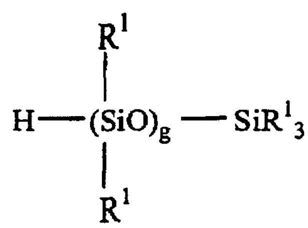

(5)

wherein $R^1$, f and g have the same meanings as in formula (3) respectively.

The organopolysiloxanes to constitute the main chains of the present silicone compounds may have a straight-chain structure or a cyclic structure. The suitable mixing ratio between a main chain polysiloxane and a silicone compound represented by formula (4) or (5) is from 0.5 to 2.0, preferably from 0.8 to 1.2, expressed in terms of the quantity by mole of the terminal unsaturated group per mole of SiH groups. The addition reaction is carried out effectively in the presence of a platinum catalyst or a rhodium catalyst. Suitable examples of such a catalyst include chloroplatinic acid, alcohol-modified chloroplatinic acid and chloroplatinic acid-vinylsiloxane complex.

The amount of catalyst used, though it may be a conventional catalytic amount, is desirably at most 500 ppm, particularly desirably at most 20 ppm, based on the platimum or the rhodium. The addition reaction may be carried out in an organic solvent, if needed. Examples of an organic solvent usable therein include aliphatic alcohols, such as methanol, ethanol, 2-propanol and butanol; aromatic hydrocarbons, such as toluene and xylene; aliphatic or alicyclic hydrocarbons, such as n-pentane, n-hexane and cyclohexane; and halogenated hydrocarbons, such as dichloromethane, chloroform and carbon tetrachloride. The addition reaction has no particular restriction as to its reaction conditions. However, it is desirable that the addition reaction be performed for 1 to 10 hours under reflux.

The present silicone compounds can be used for various purposes. In particular, they are suitable for materials

of all cosmetics applied to skin and hair. The appropriate proportion of a silicone compound represented by formula (1) mixed in such cosmetics each is from 0.1 to 80 % by weight.

The present cosmetic materials can further contain other oils depending on the desired purposes thereof. The oils used together may be in any of solid, semisolid and liquid states, provided that they have so far been used for general cosmetics.

More specifically, not only natural animal and vegetable fats and oils but also semi-synthetic fats and oils can be mixed in the present cosmetic material, with examples including avocado oil, linseed oil, almond oil, Chinese wax, perilla oil, olive oil, cacao butter, kapok wax, kaya oil, carnauba wax, liver oil, candellila wax, beef tallow, beef foot oil, beef bone fat, hydrogenated beef tallow, apricot kernel oil, spermaceti, hydrogenated oil, wheat germ oil, sesame oil, rice germ oil, rice bran oil, sugar cane wax, sasanqua oil, safflower oil, shea butter, Chinese tung oil, cinnamon oil, jojoba wax, shellac wax, turtle oil, soybean oil, tea seed oil, tsubaki oil, evening primrose oil, corn oil, lard, rape seed oil, Japanese tung oil, rice-bran wax, germ oil, horse fat, persic oil, palm oil, palm kernel oil, castor oil, hydrogenated castor oil, methyl caster oil fatty acid, sunflower oil, grape seed oil, bayberry wax, jojoba oil, macadamia nut oil, beeds wax, mink oil, cottonseed oil, cotton wax, Japan wax, haze kernel oil, montan wax, coconut oil, hydrogenated coconut oil, tricoconut oil fatty acid glyceride, mutton-tallow, peanut oil, lanolin, liquid lanolin, reduced lanolin, lanolin alcohol, hard lanolin, lanolin acetate, lanolin fatty acid isopropyl, hexyl laurate, POE lanolin alcohol ether, POE lanolin alcohol acetate, polyethylene glycol lanolin fatty acid, POE hydrogenated lanolin alcohol ether, and egg yolk oil.

Examples of hydrocarbon oil which can be mixed therein include ozokerite, squalane, squalene, ceresine, paraffin, paraffin wax, liquid paraffin, pristane, polyisobutylene, microcrystalline wax and Vaseline; and those of a higher fatty acid which can be mixed include lauric acid, myristic acid, palmitic acid, stearic acid, behenic acid, undecylenic acid, oleic acid, linoleic acid, linolenic acid, arachidonic acid, eicosapentaenoic acid (EPA), docosahexaenoic acid (DHA), isostearic acid and 12-hydroxystearic acid.

Examples of a higher alcohol which can be mixed therein include lauryl alcohol, myristyl alcohol, palmityl alcohol, stearyl alcohol, behenyl alcohol, hexadecyl alcohol, oleyl alcohol, isostearyl alcohol, hexyldodecanol, octyldodecanol, cetostearyl alcohol, 2-decyltetradecinol, cholesterol, phytosterol, POE cholesterol ether, monostearyl glycerin ether (batyl alcohol) and monooleyl glyceryl ether (cerakyl alcohol).

Examples of ester oil which can be mixed therein include diisobutyl adipate, 2-hexyldecyl adipate, di-2-heptylundecyl adipate, N-alkylglycol monoisostearates, isocetyl isostearate, trimethylolpropane triisostearic acid ester, ethylene glycol di-2-ethylhexanoic acid ester, cetyl 2-ethylhexanoate, trimethylolpropane tri-2-ethylhexanoic acid ester, pentaerythritol tetra-2-ethylhexanoic acid ester, cetyl octanoate, octyldodecyl gum ester, oleyl oleate, octyldodecyl oleate, decyl oleate, neopentyl glycol dicapric acid ester, triethyl citrate, 2-ethylhexyl cinnamate, amyl acetate, ethyl acetate, butyl acetate, isocetyl stearate, butyl stearate, diisopropyl sebacate, di-2-ethylhexyl sebacate, cetyl lactate, myristyl lactate, isopropyl palmitate, 2-ethylhexyl palmitate, 2-hexyldecyl palmitate, 2-heptylundecyl palmitate, cholesteryl 12-hydroxystearate, dipentaerythritol fatty acid esters, isopropyl myristate, octyldodecyl myristate, 2-hexyldecyl myristate, myristyl myristate, hexyldecyl dimethylocanoate, ethyl laurate, hexyl laurate, N-lauroyl-L-glutaminic acid 2-octyldodecyl ester and diisostearyl malic acid; and examples of glyceride oil which can be mixed therein include acetoglyceride, triisooctanoic acid glycride, triisostearic acid glyceride, triisopalmitic acid glyceride, tri-2-ethylhexanoic acid glyceride, monostearic acid glyceride, di-2-heptylundecanoic acid glyceride, trimyristic acid glyceride and myristic acid isostearic acid diglyceride.

As examples of other silicone oils which can be mixed, mention may be made of organopolysiloxanes having from low to high viscosities, such as dimethylpolysiloxane, methylphenylpolysiloxane, methylhydrogenpolysiloxane and dimethylsiloxane-methylphenylsiloxane copolymer; cyclic siloxanes, such as octamethylcyclotetrasiloxane, decamethylcyclopentasiloxane, dodecamethylcyclohexasiloxane, tetramethyltetrahydrogencyclotetrasiloxane; silicone rubbers, such as gummy dimethylpolysiloxanes having high polymerization degrees and gummy dimethylsiloxane-methylphenylsiloxane copolymers having high polymerization degrees; and cyclosiloxane solutions of silicone rubber, trimethylsiloxysilicate, cyclosiloxane solutions of trimethylsiloxysilicate, higher alkoxy-modified silicones such as stearoxysilicone, higher fatty acid-modified silicones, alkyl-modified silicones, amino-modified silicones, fluorine-modified silicones, and silicone resin solutions.

These silicone oils are not particularly restricted as to their structure, but they may have any of straight-chain, branched and cyclic structures. In particular, it is desirable that their main skeletons be $-[Si-O]_n-$. Additionally, those silicone oils may have $-Si-(CH_2CH_2)_m-Si-$ linkage in part of their respective molecules.

As examples of fluorine-containing oil which can be mixed, mention may be made of perfluoropolyether, perfluorodecalin and perfluorooctane.

Furthermore, the present cosmetic material can contain one or more of a surfactant, if desired. The surfactant added thereto has no particular restriction, but it may be any of anionic, cationic, nonionic and amphoteric ones so long as they have hitherto been used in general cosmetics.

Examples of a usable anionic surfactant include fatty acid soap, such as sodium stearate or triethanolamine palmitate; alkyl ether carboxylic acids and salts thereof; salts of amino acid-fatty acid condensates; alkanesulfonates;

alkenesulfonates; sulfonated fatty acid esters; sulfonated fatty acid amides; sulfonates of formaldehyde condensate type; alkylsulfates; higher secondary alcohol sulfates; alkyl and aryl ether sulfates; fatty acid ether sulfates, fatty acid alkylolamide sulfates; ether sulfates, such as Turkeky red oil; alkyl phosphates; ether phosphates; alkyl aryl ether phosphates; amide phosphates; and active agents of N-acylamino acid type.

Examples of a usable cationic surfactant include amine salts, such as alkylamie salts, polyamines and aminoalcohol fatty acid derivatives, quaternary alkylammonium salts, quaternary arylammonium salts, pyridinium salts and imidazolium salts.

Examples of a usable nonionic surfactant include sorbitan fatty acid esters, glycerin fatty acid esters, polyglycerin fatty acid esters, propylene glycol fatty acid esters, polyethylene glycol fatty acid esters, sucrose fatty acid esters, polyoxyethylene alkyl ethers, polyoxypropylene alkyl ethers, polyoxyethylene alkyl phenyl ethers, polyoxyethylene fatty acid esters, polyoxyethylene sorbitan fatty acid esters, polyoxyethylene sorbitol fatty acid esters, polyoxyethylene glycerin fatty acid esters, polyoxyethylene propylene glycol fatty acid esters, polyoxyethylene castor oil, polyoxyethylene hydrogenated castor oil, polyoxyethylene phytostanol ehter, polyoxyethylene phytosterol ether, polyoxyethylene cholestanol ether, polyoxyethylene cholesteryl ether, polyoxyalkylene-modified organopolysiloxanes, organopolysiloxanes modified with both polyoxyalkylene and alkyl groups, alkanolamides, sugar ethers and sugar amides; and examples of a usable amphoteric surfactant include betaine, aminocarboxylate and imdazoline derivatives.

Furthermore, the present cosmetic material can contain powders depending on the desired purposes. Such powders are not particularly restricted as to their shape (whether it is spherical, acicular or tabular), their size (whether it is on the order of fume, fine grain or pigment), and their structure (whether it is porous or nonporous), provided that they have so far been used in conventional cosmetic materials. For instance, inorganic powders, organic powders, surfactant metal salt powders, colored pigments, pearl pigments, metallic powder pigments and natural colors can be added to the present cosmetic material, if desired.

Examples of a usable inorganic powder include titanium oxide, zirconium oxide, zinc oxide, cerium oxide, magnesium oxide, barium sulfate, calcium sulfate, magnesium sulfate, calcium carbonate, magnesium carbonate, talc, mica, kaolin, sericite, muscovite, synthetic mica, phlogopite, ruby mica, biotite, lipidolite, silicic acid, silicic acid anhydride, aluminum silicate, magnesium silicate, aluminum magnesium silicate, calcium silicate, barium silicate, strontium silicate, metal salts of tungstic acid, hydroxyapatite, vermiculite, haidilite, bentonite, montmorillonite, hectorite, zeolite, ceramics powder, calcium secondary phosphate, alumina, aluminum hydroxie, boron nitride and silica.

Examples of a usable organic powder include polyamide powder, polyester powder, polyethylene powder, polypropylene powder, polystyrene powder, polyurethane powder, benzoguanamine powder, polymethylbenzoguanamine powder, polytetrafluoroethylene powder, polymethylmethacrylate powder, cellulose powder, silk powder, nylon powder such as 12-nylon powder or 6-nylon powder, silicone elastomer powder, styrene-acrylic acid copolymer powder, divinylbenzene-styrene copolymer powder, vinyl resin powder, urea resin powder, phenol resin powder, fluororesin powder, silicone resin powder, acrylic resin powder, melamine resin powder, epoxy resin powder, polycarbonate resin powder, microcrystalline fiber powder, starch powder and lauroyl lysine powder. In addition, the organic powders the main skeleton of which is $-[Si-O]_n-$ can be used to advantage. These silicone powders may contain $-Si-(CH_2CH_2)_m-Si-$ linkage as a part of molecular structure.

Examples of a usable surfactant metal salt powder (metal soap powder) include powders of zinc stearate, aluminum stearate, calcium stearate, magnesium stearate, zinc myristate, magensium myristate, zinc cetylphosphate, calcium cetylphosphate and zinc sodium cetylphosphate.

Examples of a usable colored pigment include inorganic red pigments, such as iron oxide, iron hydroxide and iron titanate; inorganic brown pigments, such as $\gamma$-iron oxide; inorganic yellow pigments, such as iron oxide yellow and loess; inorganic black pigments, such as iron oxide black and carbon black; inorganic violet pigments, such as manganese violet and cobalt violet; inorganic green pigments, such as chromium hydroxide, chromium oxide, cobalt oxide and cobalt titanate; inorganic blue pigments, such as Prussian blue and ultramarine blue; lakes of tar pigments; lakes of natural dyes; and synthetic resin powder complexes of the inorganic pigments as recited above.

Examples of a usable pearl pigment include titanium oxide-coated mica, bismuth oxychloride, titanium oxide-coated bismuth oxychloride, titanium oxide-coated talc, fish scales, and titanium oxide-coated colored mica; and those of a usable metallic powder pigment include aluminum powder, copper powder and stainless powder.

The tar pigments described above include Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206 and Orange No. 207 (according to the pigment nomenclature method in JIS) ; and the natural pigments described above include powders of carminic acid, laccaic acid, carthamin, bradilin and crocin.

Additionally, complexes of the powders as recited above or those obtained by treating the powders as recited above with general oil, silicone oil, a fluorine-containing compound or a surfactant may be used. Also, the powders as

recited above may be used as a mixture of two or more thereof, if desired.

In the present cosmetic materials, the compounds having at least one alcoholic hydroxyl group per molecule can be used depending on the desired purposes of the cosmetic materials.

Examples of alcohols which can be added include lower alcohols, such as ethanol and isopropanol; sugar alcohols, such as sorbitol and maltose; and sterols, such as cholesterol, sitosterol, phytosterol and lanosterol.

Examples of water-soluble polymers which can be added include vegetable polymers, such as gum arabic, tragacanth, galactan, carob gum, guar gum, karaya gum, carrageenan, pectin, agar, quince seed, starch (rice, corn, potato, wheat), alge colloid, tranto gum and locust bean gum; microbial polymers, such as xanthan gum, dextran, succinoglucan and pullulan; animal polymers, such as collagen, casein, albumin and gelatin; starch polymers, such as carboxymethyl starch and methylhydroxypropyl starch; cellulose polymers, such as methyl cellulose, ethyl cellulose, methylhydroxypropyl cellulose, carboxymethyl cellulose, hydroxymethyl cellulose, hydroxypropyl cellulose, nitrocellulose, sodium cellulose sulfate, sodium carboxymethylcellulose, crystalline cellulose and powdery cellulose; alginic acid polymers, such as sodium alginate and propylene glycol ester of alginic acid; vinyl polymers, such as polyvinyl methyl ether and carboxyvinyl polymer; polyoxyethylene polymers; polyoxyethylene-polyoxypropylene copolymers; acrylic polymers, such as sodium polyacrylate, polyethylacrylate and polyacrylamide; other synthetic water-soluble polymers, such as polyethyleneimines and cationic polymers; and inorganic water-soluble polymers, such as bentonite, aluminum magnesium silicate, montmorillonite, beidellite, nontronite, saponite, hectorite and silicic acid anhydride.

In these water-soluble polymers, film-forming agents, such as polyvinyl alcohol and polyvinyl pyrrolidine, are also included.

The present cosmetic materials can further contain cross-linked organopolysiloxanes. The cross-linked organopolysiloxanes suitable for the present cosmetic materials are those which cause swelling when they contain a silicone having low viscosity of from 0.65 to 10.0 $mm^2$/sec (25°) in a quantity larger than their self weight. Further, it is desirable that the cross-linked structure of those organopolysiloxanes be formed by the reaction between the hydrogen atoms bonded directly to silicon atoms and a cross-linking agent having at least two vinylic reactive moieties per molecule. Furthermore, it is desirable in the foregoing reaction to use the cross-linking agent containing at least one moiety selected from polyoxyalkylene, alkyl, alkenyl, aryl and fluoroalkyl moieties. The suitable proportion of such cross-linked organopolysiloxanes mixed in the present cosmetic material is from 0.1 to 30.0 weight %, preferably from 1.0 to 10.0 weight %, to the total weight of the cosmetic material.

The present cosmetic material can further contain one or more of silicone resins, such as acryl-silicone graft or block copolymers and silicone compounds having network structure, if needed.

In particular, acrylsilicone resins are suitable for the present cosmetic material. Further, it is desirable that at least one moiety selected from the group consisting of pyrrolidone, long-chain alkyl, polyoxyalkylene and fluoroalkyl moieties be present in such an acrylsilicone resin molecule.

The other favorable silicone resins are silicone compounds having network structure. When the silicone resins, such as acryl-silicone graft or block copolymer and silicone compounds having network structure, are mixed in the present cosmetic material, the appropriate proportion of silicone resins mixed is from 0.1 to 20 weight %, preferably from 1 to 10 weight %, to the total weight of the cosmetic material.

To the present cosmetic material, the ingredients used in general cosmetic materials, such as water, a film-forming agent, an oil-soluble gelling agent, clay minerals modified with organic compounds, resins, ultraviolet absorbents, a moisture-holding agent, antiseptics, an antimicrobial agent, perfume, salts, antioxidants, pH regulators, a chelating agent, refrigerant, an anti-inflammatory agent, skin beautifying components (a skin whitener, a cell activator, a rough dry skin improver, a blood circulation promoter, a skin astringent and an anti-seborrheic agent), vitamins, amino acids, nucleic acids, hormones and clathrate compounds, can be added so far as they have no adverse influence on the effects of the present invention.

Examples of an oil-soluble gelling agent which can be added include metal soaps, such as aluminum stearate, magnesium stearate and zinc myristate; amino acid derivatives, such as N-lauroyl-L-glutamic acid and $\alpha,\gamma$-di-n-butylamine; dextrin fatty acid esters, such as dextrin palmitic acid ester, dextrin stearic acid ester and dextrin 2-ethylhexaminic acid palmitic acid ester; sucrose fatty acid esters, such as sucrose palmitic acid ester and sucrose stearic acid ester; benzylidene derivatives of sorbitol, such as monobenzylidene sorbitol and dibenzylidene sorbitol; and clay minerals modified with organic compounds, such as dimethylbenzyldodecyl ammonium montmorillonite clay and dimethyldioctadecyl ammonium montmorillonite clay.

Examples of an ultraviolet absorbent which can be added include ultraviolet absorbents of benzoic acid type, such as p-aminobenzoic acid; those of anthranilic acid type, such as methyl anthranilate; those of salicylic acid type, such as methyl salicylate; those of succinic acid type, such as octyl p-methoxysuccinate; those of benzophenone type, such as 2,4-dihydroxybenzophenone; those of urocanic acid type, such as ethyl urocanate; and those of dibenzoylmethane type, such as 4-t-butyl-4'-methoxydibenzoylmethane.

Examples of a moisture-holding agent which can be added include glycerin, sorbitol, propylene glycol, dipropylene glycol, 1,3-butylene glycol, glucose, xylitol, maltitol, polyethylene glycol, hyaluromic acid, chondroitin sulfuric

acid, pyrrolidone carboxylic acid, polyoxyethylene glycoside, and polyoxypropylene methylglycoside.

Examples of an antiseptic agent which can be added include alkyl p-hydroxybenzoates, benzoic acid, sodium benzoate, sorbic acid, potassium sorbate and phenoxyethanol; and those of an antimicrobial agent which can be added include benzoic acid, salicylic acid, carbolic acid, sorbic acid, alkyl p-hydroxybenzoates, p-chlorometacresol, hexachlorophene, benzalkonium chloride, chlorhexidine chloride, trichlorocarbanilide, photosensitizer and phenoxyethanol.

Examples of an antioxidant which can be added include tocopherol, butylhydroxyanisole, dibutylhydroxytoluene and phytic acid; those of a pH regulator which can be added include lactic acid, citric acid, glycolic acid, succinic acid, tartaric acid, dl-malic acid, potassium carbonate, sodium hydrogen carbonate and ammonium hydrogen carbonate; those of a chelating agent which can be added include alanine, sodium ethylenediaminetetraacetate, sodium polyphosphate, sodium metaphosphate and phosphoric acid; those of a refrigerant which can be added include L-menthol and camphor; and those of an anti-inflammatory agent which can added include allantoin, glycyrrhizin and salts thereof, glycyrrhetinic acid and stearyl glycyrrhetinate, tranexamic acid and azulene.

Examples of a skin-beautifying component which can be added include whitening agents, such as placenta extract; arbutin, glutathione and Yukinoshita extract; cell activators, such as royal jelly, photosensitizer, cholesterol derivatives and calf blood extract; rough dry skin improvers; blood circulation improvers, such as nonylic acid vanillyl amide, benzyl nicotinate, β-butoxyethyl nicotinate, capsaicin, zingerone, cantharis tincture, ichtammol, caffeine, tannic acid, α-borneol, tocopheryl nicotinate, inositol hexanicotinate, cyclandelate, cinnarizine, tolazoline, acetyl choline, verapamil, cepharanthin and γ-oryzanol; skin astringents, such as zinc oxide and tannic acid; and anti-seborrheic agents, such as sulfur and thianthol.

Examples of vitamins which can be added include vitamin A, such as vitamin A oil, retinol, retinyl acetate and retinyl palmitate; vitamin B, including vitamin $B_2$ such as riboflavin, riboflavin butyrate and flavin adenine nucleotide, vitamin $B_6$ such as pyridoxine hydrochloride, pyridoxine dioctanoate and pyridoxine tripalmitate, vitamin $B_{12}$ and its derivatives, and vitamin $B_{15}$ and its derivatives; vitamin C, such as L-ascorbic acid, L-ascorbic acid dipalmitic ester, sodium (L-ascorbic acid)-2-sulfate and dipotassium L-ascorbic acid diphosphate; vitamin D, such as ergocalciferol and cholecarciferol; vitamin E, such as α-tocopherol, β-tocopherol, γ-tocopherol, dl-α-tocopheryl acetate, dl-α-tocopheryl nicotinate and dl-α-tocopheryl succinate; vitamin H; vitamin P; nicotinic acids, such as nicotinic acid, benzyl nicotinate and nicotinic acid amide; pantothenic acids, such as calcium pantothenate, D-pantothenyl alcohol, pantothenyl ethyl ether and acetylpantothenyl ethyl ether; and biotin.

Examples of an amino acid which can be added include glycine, valine, leucine, isoleucine, serine, threonine, phenylaranine, alginine, lysine, aspartic acid, aspartic acid, glutamic acid, cystine, cysteine, methionine, and tryptophan; those of a nucleic acid which can be added include deoxyribonucleic acid; and those of hormone which can be added include estradiol and ethenyl estradiol.

The term "cosmetic material" as used herein are intended to include skin care products, such as face lotion, milky lotion, cream, face cleansing cream, massage materials, toilet soap and detergent, antiperspirant and deodorant; makeup products, such as face powder, foundation, rouge, eye shadow, mascara, eyeliner and lipstick; and hairdressing products, such as shampoo, rinse and treatment.

Additionally, the present cosmetic material may have any of forms, including liquid, emulsion, solid, paste, gel and spray forms, if desired.

The present invention will now be illustrated in greater detail by reference to the following examples and comparative examples. However, the invention should not be construed as being limited to these examples. And the term "%" used hereinafter means "% by weight" unless noted otherwise.

SYNTHESIS EXAMPLE 1.

After 282 parts by weight of organohydrogensiloxanes represented by the following average structural formula (6) and 638 parts by weight of toluene were placed in a reaction vessel, 2 parts by weight of a 0.5 % toluene solution of chloroplatinic acid was added thereto.

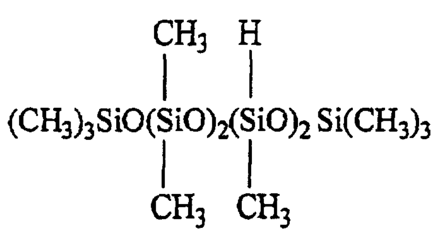 (6)

Further thereto, 356 parts by weight of pentamethyl-vinyldisiloxane was added dropwise under reflux of the solvent to undergo the reaction for 6 hours.

Then, the reaction mixture was heated under reduced pressure to distill off the solvent therefrom. Thus, organopolysiloxanes having the following average structural formula (7) were obtained.

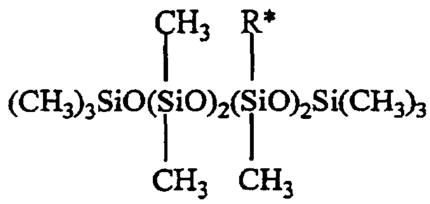 (7)

Therein, -R* represents

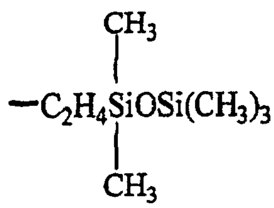 .

The product obtained was colorless transparent liquid, and it had a viscosity of 7 $mm^2/s$ (at 25°C) and a specific gravity of 0.885 (at 25°C).

<u>SYNTHESIS EXAMPLE 2</u>

After 208 parts by weight of organohydrogensiloxanes represented by the following average structural formula (8) and 638 parts by weight of toluene were placed in a reaction vessel, 2 parts by weight of a 0.5 % toluene solution of chloroplatinic acid was added thereto.

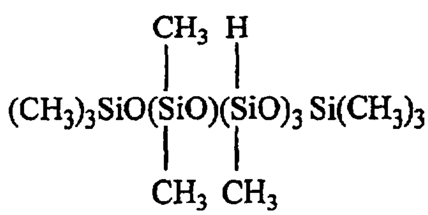 (8)

Further thereto, 1,100 parts by weight of organopolysiloxanes having the following structural formula (9) were added dropwise under reflux of the solvent to undergo the reaction for 6 hours.

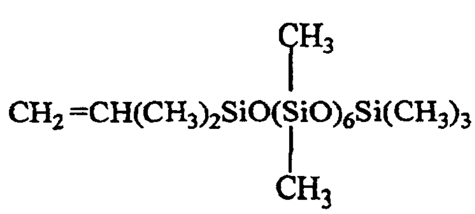 (9)

Then, the reaction mixture was heated under reduced pressure to distill off the solvent therefrom. Thus, organopolysiloxanes having the following average structural formula (10) were obtained.

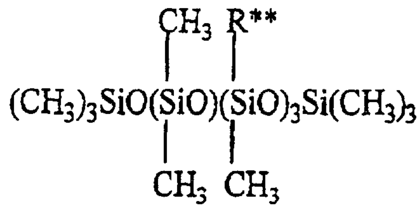

(10)

Therein, -R** represents

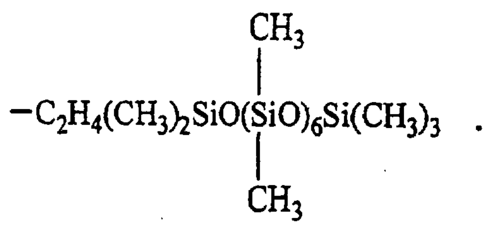

The product obtained was colorless transparent liquid, and it had a viscosity of 32 $mm^2/s$ (at 25°C) and a specific gravity of 0.947 (at 25°C).

<u>SYNTHESIS EXAMPLE 3</u>

After 293 parts by weight of organohydrogensiloxanes represented by the following average structural formula (11) and 1,300 parts by weight of toluene were placed in a reaction vessel, 2 parts by weight of a 0.5 % toluene solution of chloroplatinic acid was added thereto.

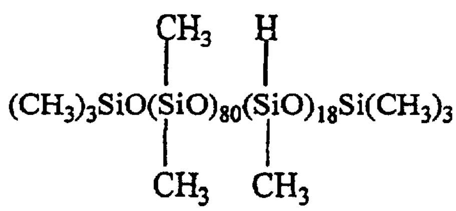

(11)

Further thereto, 1,000 parts by weight of organopolysiloxanes having the following structural formula (12) were added dropwise under reflux of the solvent to undergo the reaction for 6 hours.

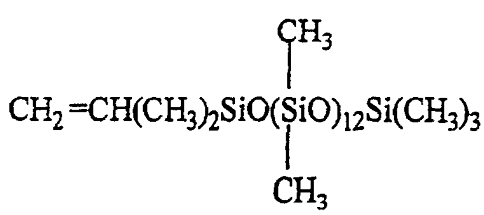

(12)

Then, the reaction mixture was heated under reduced pressure to distill off the solvent therefrom. Thus, organopolysiloxanes having the following average structural formula (13) were obtained.

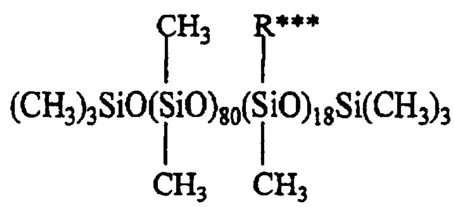

(13)

Therein, R*** represents

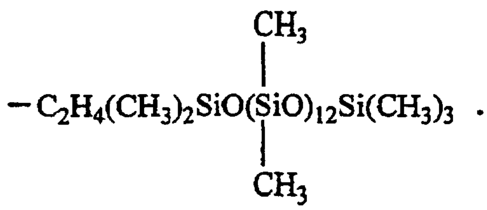

The product obtained was colorless transparent liquid, and it had a viscosity of 300 $mm^2/s$ (at 25°C) and a specific gravity of 0.963 (at 25°C).

<u>SYNTHESIS EXAMPLE 4</u>

After 409 parts by weight of organohydrogensiloxanes represented by the following average structural formula (14) and 490 parts by weight of toluene were placed in a reaction vessel, 2 parts by weight of a 0.5 % toluene solution of chloroplatinic acid was added thereto.

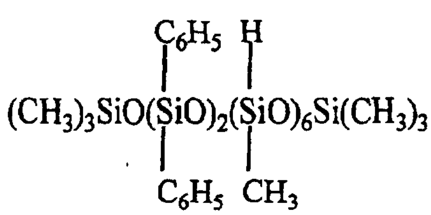

(14)

Further thereto, 530 parts by weight of pentamethyl-vinyldisiloxane was added dropwise under reflux of the solvent to undergo the reaction for 6 hours.

Then, the reaction mixture was heated under reduced pressure to distill off the solvent therefrom. Thus, organopolysiloxanes having the following average structural formula (15) were obtained.

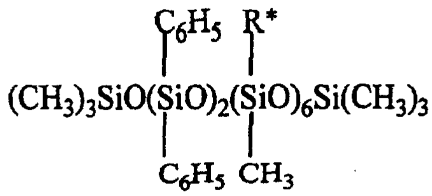

(15)

Therein, -R* has the same meaning as in Synthesis Example 1.

The product obtained was colorless transparent liquid, and it had a viscosity of 230 $mm^2/s$ (at 25°C) and a specific gravity of 0.977 (at 25°C).

SYNTHESIS EXAMPLE 5

After 546 parts by weight of organohydrogensiloxanes represented by the following average structural formula (16) and 360 parts by weight of toluene were placed in a reaction vessel, 0.2 parts by weight of a 2 % toluene solution of chloroplatinic acid was added thereto.

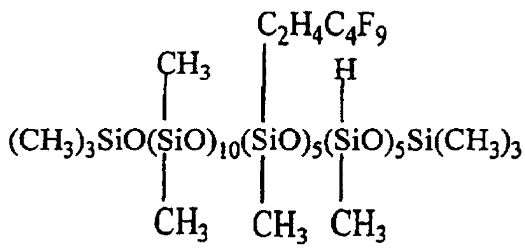

(16)

Further thereto, 174 parts by weight of pentamethyl-vinyldisiloxane was added dropwise under reflux of the solvent to undergo the reaction.

Then, the reaction mixture was heated under reduced pressure to distill off the solvent therefrom. Thus, organopolysiloxanes having the following average structural formula (17) were obtained.

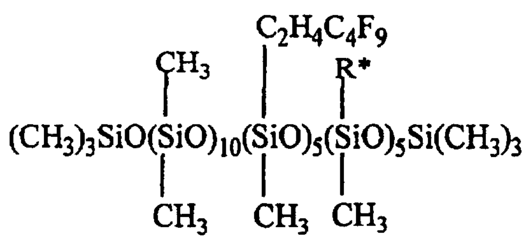

(17)

Therein, R* has the same meaning as in Synthesis Example 1.

The product obtained was colorless transparent liquid, and it had a viscosity of 113 $mm^2/s$ (at 25°C) and a specific gravity of 1.126 (at 25°C).

SYNTHESIS EXAMPLE 6

After 268 parts by weight of cyclosiloxanes represented by the following average structural formula (18) and 300 parts by weight of toluene were placed in a reaction vessel, 0.2 parts by weight of a 2 % toluene solution of chloroplatinic acid was added thereto.

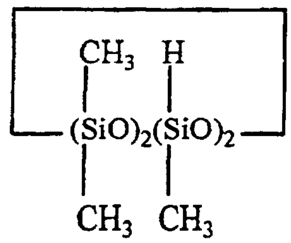

(18)

Further thereto, 348 parts by weight of pentamethyl-vinyldisiloxane was added dropwise under reflux of the solvent to undergo the reaction.

Then, the reaction mixture was heated under reduced pressure to distill off the solvent therefrom. Thus, organopolysiloxanes having the following average structural formula (19) were obtained.

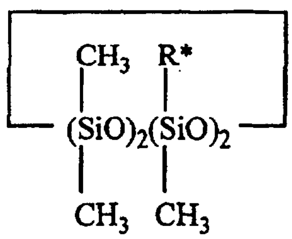

(19)

Therein, R* has the same meaning as in Synthesis Example 1.

The product obtained was colorless transparent liquid, and it had a viscosity of 12 $mm^2/s$ (at 25°C) and a specific gravity of 0.930 (at 25°C).

SYNTHESIS EXAMPLE 7

In a reaction vessel were placed 200 parts by weight of methylvinylpolysiloxanes having a vinyl value of 0.135 mole/100g, 920 parts by weight of organopolysiloxanes having the following average structural formula (20) and 1,000 parts by weight of toluene. Further thereto was added 2 parts by weight of 0.5 % of toluene solution of chloroplatinic acid. Therein, the reaction was carried out for 6 hours under reflux of the solvent. Then, the reaction mixture was heated under reduced pressure to distill off the solvent therefrom. Thus, silicone-grafted organopolysiloxanes were obtained. This product was colorless transparent liquid, and it had a viscosity of 1,800 $mm^2/s$ (at 25°C) and a specific gravity of 0.968 (at 25°C).

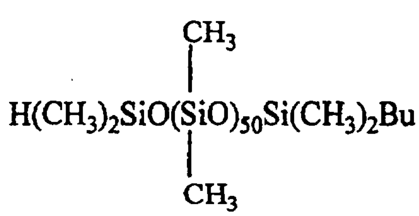

(20)

SYNTHESIS EXAMPLE 8

In a reaction vessel, 1,000 parts by weight of the silicone-grafted organopolysiloxanes obtained in Synthesis Example 7 and 200 parts by weight of organopolysilixane gum were placed, and mixed with stirring. Thus, silicone gum solution having a viscosity of 40,000 mPa·s (25°C) was obtained.

SYNTHESIS EXAMPLE 9

In a reaction vessel, 700 parts by weight of a toluene solution of silicone resin having a weight average molecular weight of 4,700 (the resin concentration was 72 %) and 520 parts by weight of the silicone-grafted organopolysiloxanes obtained in Synthesis Example 6 were placed. The contents in the vessel were heated up to 120°C under reduced pressure, thereby distilling off the toluene therefrom. Further, the temperature insides the vessel was raised to 130°C to distill off 30 parts by weight of the silicone-grafted organopolysiloxane, and to thoroughly distill off the toluene. After filtration and subsequent adjustment of volatile components, 1,000 parts by weight of a 50 % siloxane solution of silicone resins was obtained.

SYNTHESIS EXAMPLE 10

After 715 parts by weight of organohydrogensiloxanes represented by the following average structural formula (21) and 700 parts by weight of toluene were placed in a reaction vessel, 2 parts by weight of a 0.5 % toluene solution of chloroplatinic acid was added thereto.

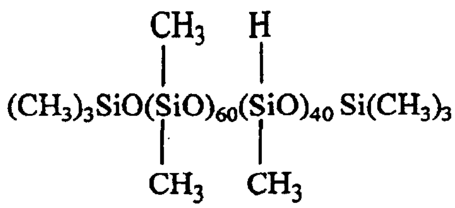

(21)

Further thereto, 174 parts by weight of pentamethyl-vinyldisiloxane was added dropwise under reflux of the solvent to undergo the reaction. Furthermore, 252 parts by weight of 1-hexene was added dropwise thereto. And the reaction was continued for 6 hours.

Then, the reaction mixture was heated under reduced pressure to distill off the solvent therefrom. Thus, organopolysiloxanes having the following average structural formula (22) were obtained.

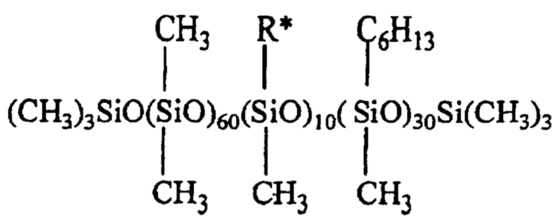

(22)

Therein, -R* has the same meaning as in Synthesis Example 1.

The product obtained was colorless transparent liquid, and it had a viscosity of 500 $mm^2/s$ (at 25°C) and a specific gravity of 0.945 (at 25°C).

SYNTHESIS EXAMPLE 11

After 480 parts by weight of organohydrogensiloxanes represented by the following average structural formula (23) and 1,000 parts by weight of toluene were placed in a reaction vessel, 0.2 parts by weight of a 2 % toluene solution of chloroplatinic acid was added thereto.

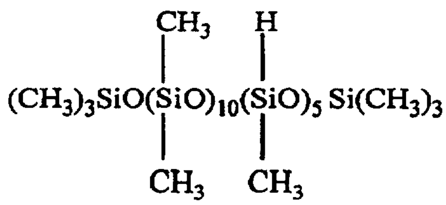

(23)

Further thereto, 600 parts by weight of the organopolysiloxanes represented by the foregoing average structural formula (9) was added dropwise under reflux of the solvent to undergo the reaction for 2 hours. Furthermore, 780 parts by weight of a 50 % toluene solution of allylstearate was added dropwise thereto. And the reaction was continued for 3 hours.

Then, the reaction mixture was heated under reduced pressure to distill off the solvent therefrom. Thus, organopolysiloxanes having the following average structural formula (24) were obtained.

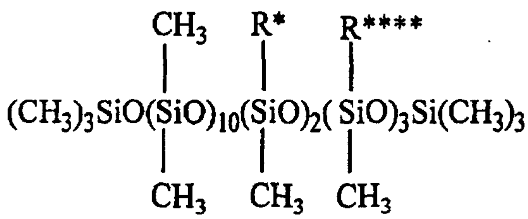

(24)

Therein, -R* has the same meaning as in Synthesis Example 1, and -R**** stands for $-C_3H_6OC(=O)C_{17}H_{35}$.

The product obtained was colorless transparent liquid, and it had a viscosity of 70 $mm^2/s$ (at 25°C) and a specific gravity of 0.947 (at 25°C).

EXAMPLES 1 TO 2 AND COMPARATIVE EXAMPLES 1 TO 5

The ingredients Nos. 1 to 11 set forth in Table 1 were each heated and mixed together to be made into a homogeneous solution. Therein, the ingredients Nos. 12 to 14 set forth in Table 1 were further dispersed homogeneously. Then, the ingredients Nos. 15 to 18 were added thereto, and the composition obtained was packed in cases. Thus, lipstick samples were produced.

Table 1

| No. | Ingredient (weight %) | Example | | Comparative Example | | | | |
|---|---|---|---|---|---|---|---|---|
| | | 1 | 2 | 1 | 2 | 3 | 4 | 5 |
| 1 | Polyethylene-polypropylene copolymer | 5 | 5 | 5 | 5 | 5 | 5 | 5 |
| 2 | Candelilla wax | 5 | 5 | 5 | 5 | 5 | 5 | 5 |
| 3 | Ceresin wax | 5 | 5 | 5 | 5 | 5 | 5 | 5 |
| 4 | Micocrystalline wax | - | - | - | 5 | 10 | - | - |
| 5 | Vaseline | 10 | 10 | 10 | 10 | 10 | 10 | 10 |
| 6 | Cetyl 2-ethylhexanoate | 16 | 11 | 16 | 11 | 16 | 11 | 16 |
| 7 | Glyceryl trioctanoate | 35 | 35 | 35 | 35 | 35 | 35 | 35 |
| 8 | Glyceryl triisostearate | 5 | 5 | 5 | 5 | 5 | 5 | 5 |
| 9 | Dimethylpolysiloxane (6 $mm^2/s$) | - | - | 5 | 10 | - | - | - |
| 10 | Fluorine-modified silicone(*) | - | - | - | - | - | - | 5 |
| 11 | Grafted silicone of Synthesis Example 5 | 5 | 10 | - | - | - | - | - |
| 12 | Pigment | 5 | 5 | 5 | 5 | 5 | 5 | 5 |
| 13 | Silicone-treated titanium oxide | 4 | 4 | 4 | 4 | 4 | 4 | 4 |
| 14 | Silicone-treated titanium mica | 5 | 5 | 5 | 5 | 5 | 5 | 5 |
| 15 | Beautifying component | Respectively proper amounts | | | | | | |
| 16 | Ultraviolet absorbent | | | | | | | |
| 17 | Antiseptic | | | | | | | |
| 18 | Perfume | | | | | | | |

(*) Fluorine-modified silicone:

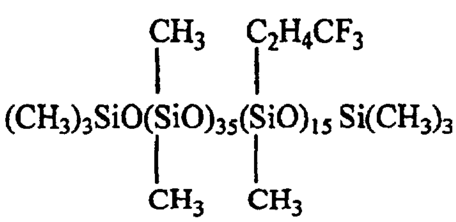

The ease-of-use, luster, makeup durability and smear resistance of the lipstick samples obtained were evaluated by 50 female panelists in accordance with the criteria shown in Table 2.

Table 2

| Marks | Ease-of-use Makeup durability | Spread | Smear resistance |
|---|---|---|---|
| 5 | good | light | good |
| 4 | fairly good | fairly light | Fairly good |
| 3 | average | average | average |
| 2 | rather bad | rather heavy | rather poor |
| 1 | bad | heavy | poor |

Judging from the average of marks given by the panelists, the quality of lipstick prepared in each Example was assessed in accordance with the standards described below:

<Assessment Standards for Average Mark>

| <Assessment Standards for Average Mark> | |
|---|---|
| Average mark obtained | Symbol (Quality) |
| 4.5 or above | ◎ (good) |
| from 3.5 to lower than 4.5 | ○ (fairly good) |
| from 2.5 to lower than 3.5 | Δ (average) |
| from 1.5 to lower than 2.5 | X (rather bad) |
| lower than 1.5 | XX (bad) |

The evaluation results according to the aforementioned assessment standards are shown in Table 3.

Table 3

| Item | Example | | Comparative Example | | | | |
|---|---|---|---|---|---|---|---|
| | 1 | 2 | 1 | 2 | 3 | 4 | 5 |
| Ease-of-use | ◎ | ◎ | ○ | ○ | × | ×× | Δ |
| Spread | ◎ | ◎ | ○ | ○ | Δ | ×× | ○ |
| Smear resistance | ○ | ○ | × | ×× | Δ | ○ | Δ |
| Makeup durability | ○ | ○ | × | ×× | Δ | ○ | ○ |

As can be seen from Table 3, it has been demonstrated that the lipsticks prepared in Examples 1 and 2 spread well and had satisfactory usability, high smear resistance and good makeup durability, compared with those prepared in Comparative Examples 1 to 5.

EXAMPLE 3

Milky lotion containing the following ingredients was prepared:

| Ingredients | | Amount mixed (%) |
|---|---|---|
| 1. | N-acylglutamic acid | 0.1 |
| 2. | 1,3-Butylene glycol | 0.4 |
| 3. | Glycerol mono fatty acid ester | 0.5 |
| 4. | Cetanol | 0.5 |
| 5. | Spermaceti | 1.0 |
| 6. | Glyceryl trioctanoate | 8.0 |

(continued)

| Ingredients | | Amount mixed (%) |
|---|---|---|
| 7. | Grafted silicone of Synthesis Example 3 | 6.0 |
| 8. | Sodium hydroxide | 0.025 |
| 9. | 1,3-Butylene glycol | 15.0 |
| 10. | Purified water | the rest |

[Preparation Process]

The milky lotion was prepared following the steps A to C described below:

(A) The ingredients 1 and 2 were mixed together under heating to prepare a solution.
(B) The solution was admixed with the ingredients 3 to 7, and this mixture was kept at 75°C.
(C) The ingredients 8 to 10 were mixed together and dissolved homogeneously by heating at 75°C. This solution was added to the foregoing mixture kept at 75°C, and emulsified, and then cooled to prepare milky lotion.

It has been confirmed that the thus prepared milky lotion not only formed a water-repellent film but also created a feeling of being moisturized when applied to the skin, and besides, it provided a pleasant feel and ease of use to the users.

EXAMPLE 4

The following ingredients were mixed together, and made into compressed face powder in accordance with the process described below:

| Ingredients | | Amount mixed (%) |
|---|---|---|
| 1. | Silicone-treated rutile-type titanium oxide | 10.0 |
| 2. | Silicone-treated anatase-type titanium oxide | 2.0 |
| 3. | Silicone-treated sericite (oil treatment with vaseline) | 35.0 |
| 4. | Lecithin-treated talc | 35.1 |
| 5. | Lecithin-treated spherical nylon powder | 5.0 |
| 6. | Iron oxide red | 0.4 |
| 7. | Iron oxide yellow | 2.0 |
| 8. | Amber | 0.4 |
| 9. | Iron oxide black | 0.1 |
| 10. | Grafted silicone of Synthesis Example 4 | 7.0 |
| 11. | Glyceryl trioctanoate | 1.5 |
| 12. | Dipentaerythritol fatty acid ester | 1.5 |

[Preparation Process]

The compressed face powder was prepared following the steps A and B described below:

(A) The ingredients 1 to 9 were mixed together, and then ground to homogeneous powder A.
(B) The powder A was admixed with the ingredients 10 to 12, ground to homogeneous powder, and then compressed tightly into a metal pan.

The face powder thus compressed spread lightly and smoothly, stayed well due to its good adhesion and was highly resistant to sweat. Thus, the present face powder can produce makeup effect of long duration, and provide a pleasant feel and excellent usability to the users.

EXAMPLE 5

An overcoat composition constituted of the following ingredients was prepared:

| Ingredients | | Amount mixed (%) |
|---|---|---|
| 1. | Polyethylene/polypropylene copolymer | 10 |
| 2. | Ceresin wax | 10 |
| 3. | Glyceryl trioctanoate | 30 |
| 4. | Grafted silicone of Synthesis Example 2 | 30 |
| 5. | Decamethylcyclopentasiloxane | 20 |

[Preparation Process]

The overcoat composition was prepared following the steps A and B described below:

(A) The ingredients 1 to 5 were mixed together to prepare a homogeneous solution.
(B) The solution obtained was charged into vessels.

It was confirmed that the thus prepared overcoat composition spread well, had excellent usability, extended the duration of makeup effect, and provided lasting moisture and luster.

EXAMPLE 6

A makeup remover constituted of the following ingredients was prepared:

| Ingredients | | Amount mixed (%) |
|---|---|---|
| 1. | Grafted silicone of Synthesis Example 1 | 20.0 |
| 2. | Polyoxyethylene(20) sorbitan monostearate | 10.0 |
| 3. | Sorbitol | 10.0 |
| 4. | Carrageenan | 0.5 |
| 5. | Antiseptic | proper |
| 6. | Perfume | proper |
| 7. | Purified water | rest |

[Preparation Process]

The makeup remover was prepared following the steps A and B described below:

(A) The ingredients 1 to 5 and 7 were mixed together to prepare a homogeneous solution.
(B) The ingredient 6 was added to the solution.

The thus prepared makeup remover was used for removing durable face powder. As a result, it was found that the face powder and the sebum stains absorbed this remover well and they came out easily. Further, the remover spread well and had no sticky feel when applied to the face, and left a refreshed feel to the face after it was wiped off. In other words, this remover had excellent usability and provided a pleasant feel to the users. In addition, it has proved that the remover caused no change by fluctuation in temperature and passage of time, namely it had high stability.

EXAMPLE 7

A hair makeup remover constituted of the following ingredients was prepared:

| Ingredients | | Amount mixed (%) |
|---|---|---|
| 1. | Grafted silicone of Synthesis Example 5 | 10.0 |
| 2. | Diethylene glycol monoethyl ether | 5.0 |
| 3. | Glycerin | 30.0 |
| 4. | Carrageenan | 0.5 |
| 5. | Antiseptic | proper |

(continued)

| Ingredients | | Amount mixed (%) |
|---|---|---|
| 6. | Perfume | proper |
| 7. | Purified water | rest |

[Preparation Process]

The hair-makeup remover was prepared following the steps A and B described below:

(A) The ingredients 1 to 5 and 7 were mixed together to prepare a homogeneous solution.
(B) The ingredient 6 was added to the solution.

The hair was washed with the thus prepared hair-makeup remover. As a result, it was found that the hair makeup and the sebum stains absorbed this remover well and they came out easily. Further, the remover spread well when applied to the hair, and had no sticky feel but left a refreshed feel to the users after it was washed out. In other words, this remover had excellent usability and provided a pleasant feel to the users. In addition, it was confirmed that the remover caused no change by fluctuation in temperature and passage of time, namely it had high stability.

EXAMPLE 8

Hair cream constituted of the following ingredients was prepared:

| Ingredients | | Amount mixed (%) |
|---|---|---|
| 1. | Silicone gum solution of Synthesis Example 8 | 18 |
| 2. | Silicone resin solution of Synthesis Example 9 | 6 |
| 3. | Glyceryl tri-2-ethylhexanoate | 8 |
| 4. | Vaseline | 5 |
| 5. | Stearyl alcohol Stearyl alcohol | 2 |
| 6. | Sorbitan monooleate | 2 |
| 7. | Polyoxyethylene (50) hydrogenated castor oil | 2 |
| 8. | Glycerol | 5 |
| 9. | Perfume | proper |
| 10. | Purified water | the rest |

[Preparation Process]

The hair cream was prepared following the steps A and B described below:

(A) The ingredients 1 to 7 were mixed together, and made into a solution by heating at 70°C.
(B) The ingredients 8 to 10 were mixed together with stirring, and thereto the solution obtained in the step A was added, followed by emulsification.

The thus prepared hair cream was found to give fine luster and smoothness to the hair and have excellent setting effect on the hair.

EXAMPLE 9

Lipstick containing the following ingredients was prepared:

| Ingredients | | Amount mixed (%) |
|---|---|---|
| 1. | Polyethylene wax | 15 |
| 2. | Candelilla wax | 3 |
| 3. | Microcrystalline wax | 2 |

(continued)

| Ingredients | | Amount mixed (%) |
|---|---|---|
| 4. | Pentaerythritol stearate | 5 |
| 5. | Cetyl isooctanoate | 31 |
| 6. | Propylene glycol didecanoate | 17 |
| 7. | Grafted silicone of Synthesis Example 10 | 27 |
| 8. | Pigment | 0.5 |

[Preparation Process]

The lipstick was prepared following the steps A and B described below:

(A) The ingredients 1 to 7 were mixed together at 90°C.
(B) Thereto, the ingredient 8 was added, and dispersed homogeneously. Then, the composition obtained was poured into cases, and cooled.

It has been shown that the thus prepared lipstick spread well and had satisfactory usability, high smear resistance and good makeup durability.

EXAMPLE 10

Skin cream constituted of the following ingredients was prepared:

| Ingredients | | Amount mixed (%) |
|---|---|---|
| 1. | Grafted silicone of Synthesis Example 11 | 3.0 |
| 2. | Grafted silicone of Synthesis Example 6 | 5.0 |
| 3. | Cetanol | 6.0 |
| 4. | Liquid paraffin | 2.0 |
| 5. | Palmitic acid | 1.0 |
| 6. | Squalane | 1.0 |
| 7. | Dimethylsilicone (viscosity: 50 $mm^2/s$) | 1.0 |
| 8. | Sorbitan monosteate | 2.0 |
| 9. | Sodium laurylpolyoxyethylene (4EO) sulfate | 1.0 |
| 10. | Glycerin | 10.0 |
| 11. | Purified water | the rest |

[Preparation Process]

The hair cream was prepared following the steps A to C described below:

(A) The ingredients 1 to 9 were mixed together, and made into a solution.
(B) The ingredients 10 and 11 were mixed together, and made into a solution.
(C) The solution obtained in the step B was added gradually to the solution obtained in the step A, followed by emulsification.

It has been shown that the thus prepared emulsion had excellent storage stability, a moist feel and high usability, and gave a feeling of comfort to the users.

EXAMPLE 11

A transparent gel antiperspirant having the following composition was prepared:

| Ingredients | | Amount mixed (%) |
|---|---|---|
| 1. | KSG-21 | 8.0 |
| 2. | Grafted silicone of Synthesis Example 1 | 12.0 |
| 3. | Dipropylene glycol | 16.0 |
| 4. | Aluminum zirconium tetrachlorohydrex (GLY) | 20.0 |
| 5. | Water | 44.0 |
| KSG-21: Cross-linked polyether-modified methylpolysiloxane/dimethylpolysiloxane copolymer | | |

[Preparation Process]

The transparent antiperspirant gel was prepared following the steps A to D described below:

(A) The ingredients 1 and 2 were mixed homogeneously.
(B) The ingredient 3 was mixed homogeneously with the mixture obtained in the step A.
(C) The ingredients 4 and 5 were mixed homogeneously.
(D) The mixture obtained in the step C was gradually added to the mixture obtained in the step B with stirring.

The thus obtained transparent antiperspirant gel spread smoothly, had neither tacky nor oily feel but a moist and fresh feel, gave a refreshing feeling to the users, and caused no change in quality by temperature and aging. Further, the duration of its effect was long. In other words, the transparent antiperspirant gel according to the invention had excellent usability and stability.

EXAMPLE 12

Foundation cream having the following composition was prepared:

| Ingredients | | Amount mixed (%) |
|---|---|---|
| 1. | KSG-21 | 7.0 |
| 2. | Glyceryl tri(2-ethylhexanoate) | 3.0 |
| 3. | Grafted silicone of Synthesis Example 1 | 8.0 |
| 4. | Sodium chloride | 0.5 |
| 5. | 1,3-Butylene glycol | 7.0 |
| 6. | KSP-100 | 1.5 |
| 7. | Pigment (treated so as to have hydrophobicity) | 10.0 |
| 8. | KSP-545 | 5.0 |
| 9. | Purified water | 58.0 |
| KSP-21: Cross-linked polyether-modified methylpolysiloxane/dimethylpolysiloxane copolymer<br>KSP-100: Cross-linked silicone-netted silicone block ccopolymer<br>KSP-545: Alkyl acrylate copolymer methylpolysiloxane ester/decamethylcyclopentasiloxane mixture | | |

[Preparation Process]

The foundation cream was prepared following the steps A to D described below:

(A) The ingredients 1 to 3 were mixed homogeneously.
(B) The ingredient 4 was dissolved in the ingredient 9, and then added to the mixture obtained in the step A, followed by emulsification.
(C) The ingredient 6 was dispersed in the ingredient 5, and then admixed with the emulsion obtained in the step B.
(D) The ingredient 7 was dispersed in the ingredient 7, and then admixed with the mixture obtained in the step C.

The thus prepared foundation cream had a light touch, formed a film having no tacky feel but a smooth feel, and ensured a long duration of makeup effect.

EXAMPLE 13

A sunscreen water-in-oil emulsion having the following composition was prepared:

| Ingredients | | Amount mixed (%) |
|---|---|---|
| 1. | Titanium dioxide (treated with stearic acid) | 20.0 |
| 2. | KP-571 | 5.0 |
| 3. | Grafted silicone of Synthesis Example 6 | 28.0 |
| 4. | Glyceryl tri(2-ethylhexanoate) | 5.0 |
| 5. | Grafted silicone of Synthesis Example 1 | 4.0 |
| 6. | 2-Ethylhexyl paramethoxycinnamate | 5.0 |
| 7. | KP-6017 | 1.5 |
| 8. | KP-6026 | 1.0 |
| 9. | 1,3-Butylene glycol | 4.0 |
| 10. | Purified water | 26.5 |

KP-571: Vinylpyrrolidone/methacryl-modified methylpolysiloxane copolymer
KP-6017: Polyoxyethylene/methylpolysiloxane copolymer
KP-6026: Polyoxyethylenemethylsiloxane/polyoxypropyleneoleylmethylsiloxane/dimethylsiloxane terpolymer

[Preparation Process]

The preparation of the above sunscreen water-in-oil emulsion was carried out following the steps A to C described below:

(A) A part of the ingredient 3 and the ingredients 4 to 8 were mixed homogeneously.
(B) The ingredient 9 was dissolved in the ingredient 10, and then added to the mixture obtained in the step A, followed by emulsification.
(C) The ingredients 1 and 2 and the remainder of the ingredient 3 were mixed and dispersed, and further admixed with the emulsion obtained in the step B.

Although a large amount of powder was mixed in the sunscreen water-in-oil emulsion thus prepared, the emulsion had low viscosity, and didn't render the emulsion-applied skin white. Further, the emulsion had excellent adhesiveness although the dispersant used therein was liquid oil, and formed a soft and natural sunscreen coating.

EXAMPLE 14

A sunscreen water-in-oil emulsion having the following composition was prepared:

| Ingredients | | Amount mixed (%) |
|---|---|---|
| 1. | Grafted silicone of Synthesis Example 6 | 15.0 |
| 2. | Methylphenylpolysiloxane | 5.0 |
| 3. | Squalane | 5.0 |
| 4. | Pentaerythritol tetra(2-ethylhexanoate) | 5.0 |
| 5. | Polyether-modified silicone[1)] | 3.0 |
| 6. | Organopolysiloxane elastomer spherical powder[2)] | 2.0 |
| 7. | Hydrophobic silica[3)] | 0.5 |
| 8. | Magnesium ascorbate phosphate | 1.0 |
| 9. | Sodium chloride | 1.0 |
| 10. | Polyethylene glycol 11000 | 1.0 |
| 11. | Propylene glycol | 8.0 |

1): KF-6017 (produced by Shin-etsu Chemical Co., Ltd.)
2): KMP594 (produced by Shin-etsu Chemical Co., Ltd.)
3): Aerosil R972 (produced by Noppon Aerosil Co., Ltd.)

(continued)

| Ingredients | | Amount mixed (%) |
|---|---|---|
| 12. | Antiseptic | proper |
| 13. | Perfume | proper |
| 14. | Purified water | rest |

[Preparation Process]

The preparation of the above emulsion was carried out following the steps A to C described below:

(A) The ingredients 1 to 5 were mixed homogeneously, and thereto the ingredients 6 and 7 was added, and further made into a homogeneous dispersion.
(B) The ingredients 8 to 10 were added to the ingredient 14, and thereto the homogeneous mixture of the ingredients 11 and 12 was added.
(C) The mixture obtained in the step B was added gradually to the dispersion obtained in the step A to make an emulsion. After the emulsion was cooled, the ingredient 13 was added thereto.

The thus prepared emulsion spread smoothly, had no tacky feel but a dry feel, and caused no change in quality by temperature and aging. In other words, it was confirmed that the present emulsion had excellent usability and stability.

EXAMPLE 15

Eye wrinkle cream having the following composition was prepared:

| Ingredients | | Amount mixed (%) |
|---|---|---|
| 1. | Grafted silicone of Synthesis Example 6 | 20.0 |
| 2. | Timethylsiloxysilicate | 5.0 |
| 3. | Polyether-modified siloxane1) | 2.0 |
| 4. | Polyether- and oleyl-modified silicone2) | 5.0 |
| 5. | Sodium chondroitin sulfate | 2.0 |
| 6. | Sodium lactate | 1.0 |
| 7. | Glycerin | 50.0 |
| 8. | Antiseptic | proper |
| 9. | Antioxidant | proper |
| 10. | Perfume | proper |
| 11. | Purified water | rest |

1): KF-6017 (produced by Shin-etsu Chemical Co., Ltd.)
2): KF-6026 (produced by Shin-etsu Chemical Co., Ltd.)

[Preparation Process]

The preparation of the above eye wrinkle cream was carried out following the steps A to C described below:

(A) The ingredients 1 to 4 and the ingredient 9 were mixed under heating.
(B) The ingredients 5 to 8 and the ingredient 11 were mixed under heating, and thereby a solution was made.
(C) The solution obtained in the step B was added gradually to the mixture obtained in the step A with stirring, thereby preparing an emulsion. After the emulsion was cooled, the ingredient 10 was added thereto.

The thus prepared eye wrinkle cream spread lightly, had neither tacky nor oily feel but a moist and fresh feel, gave a refreshing feeling to the users, and caused no change in quality by temperature and aging. In other words, the eye wrinkle cream according to the invention had excellent usability and stability.

ADVANTAGES OF THE INVENTION

When the cosmetics in which the present silicone compounds represented by formula (1) are mixed are put on the skin, they spread smoothly, have no oily feel, and render the skin moist, fresh and youthful. Further, they provide a refreshed feel and durable makeup effect to the users, and besides, cause no change by fluctuation of temperature and passage of time, namely have very high stability. When the present silicone compounds are mixed in skin cleansing compositions, on the other hand, the resulting compositions acquire a characteristic that they absorb cosmetics and sebum stains very well and remove them efficiently in addition to having the foregoing characteristics, such as use comfort, excellent usability and high stability to aging.

## Claims

**1.** A cosmetic material containing a silicone compound represented by the following formula (1):

$$R^1{}_aR^2{}_bSiO_{(4-a-b)/2} \qquad (1)$$

wherein $R^1$ groups, which are the same or different, each represent a hydrogen atom or an organic group selected from the class consisting of alkyl groups containing 1 to 30 carbon atoms, aryl groups, aralkyl groups, fluorinated alkyl groups and organic groups represented by the following formula (2); $R^2$ groups each represent a silicone group represented by the following formula (3); a is a number of from 1.0 to 2.5; b is a number of from 0.001 to 1.5;

$$-C_cH_{2c}-O-(C_2H_4O)_d(C_3H_6O)_eR^3 \qquad (2)$$

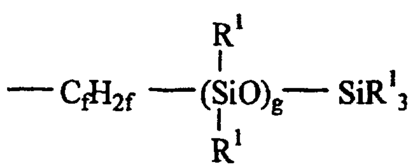

(3)

wherein $R^3$ is a hydrocarbon group containing 4 to 30 carbon atoms, or an organic group represented by $R^4$-(CO)-; $R^4$ is a hydrocarbon group containing 1 to 30 carbon atoms; c is an integer of from 0 to 15, d is an integer of from 0 to 50, and e is an integer of from 0 to 50; and f is an integer of from 1 to 5, and g is an integer of from 0 to 500.

**2.** A cosmetic material according to claim 1; further containing oils as a constituent.

**3.** A cosmetic material according to claim 2, wherein at least one of the oils is in a liquid state at room temperature.

**4.** A cosmetic material according to claim 2; wherein all or at least one of the oils is a silicone oil containing volatile silicones, a silicone oil having constitutional repeating units represented by $-[O-Si]_n-$ in its molecular skeleton, or a mixture thereof.

**5.** A cosmetic material according to claim 2, 3 or 4; wherein all or at least one of the oils is an oil having fluorine-containing or amino groups.

**6.** A cosmetic material according to any of claims 1 to 5; further containing at least one ingredient selected from the group consisting of surfactants, powders and coloring materials.

**7.** A cosmetic material according to claim 6, wherein the surfactants are modified silicones having polyoxyalkylene chains.

**8.** A cosmetic material according to claim 6 or 7, wherein the surfactants are compounds having their HLB values in

the range of 2 to 8.

9. A cosmetic material according to any of claims 6 to 8, wherein all or a part of the powder, the coloring material or the mixture thereof is an organic powder constituted of a silicone resin powder, a powder having a silicone elastomer as its skeleton, an organic powder containing constitutional repeating units represented by $-[O\text{-}Si]_n-$ or a mixture of two or more thereof.

10. A cosmetic material according to claim 1; further containing water as a constituent.

11. A cosmetic material according to any of claims 1 to 10; further containing as a constituent a compound having an alcoholic hydroxyl group in its molecular structure.

12. A cosmetic material according to claim 11, wherein the compound having an alcoholic hydroxyl group in its molecular structure is a monohydric water-soluble alcohol, a polyhydric water-soluble alcohol or a mixture thereof.

13. A cosmetic material according to claim 11, wherein the compound having an alcoholic hydroxyl group in its molecular structure is a water-soluble polymer.

14. A cosmetic material according to any of claims 1 to 13; further containing cross-linked organopolysiloxanes as a constituent.

15. A cosmetic material according to claim 14, wherein the cross-linked organopolysiloxanes are cross-linked organopolysiloxanes which cause swelling when they contain a silicone having low viscosity of from 0.65 to 10.0 $mm^2/sec$ (25°) in a quantity larger than their self weight.

16. A cosmetic material according to claim 14 or 15, wherein the cross-linked organopolysiloxanes are organopolysiloxanes having a cross-linked structure formed by the reaction between the hydrogen atoms bonded directly to silicon atoms and a cross-linking agent having at least two vinylic reactive moieties per molecule.

17. A cosmetic material according to any of claims 14 to 16, wherein the cross-linked organopolysiloxanes are organopolysiloxanes having in their cross-links at least one kind of moiety selected from the family consising of polyoxyalkylene, alkyl, alkenyl, aryl and fluoroalkyl moieties.

18. A cosmetic material according to any of claims 1 to 17; further containing silicone resin as a constituent.

19. A cosmetic material according to claim 18, wherein the silicone resin is an acrylsilicone resin.

20. A cosmetic material according to claim 18 or 19, wherein the silicone resin is an acrylsilicone resin containing at least one moiety selected from the group consisting of pyrrolidone, long-chain alkyl, polyoxyalkylene and fluoroalkyl moieties.

21. A cosmetic material according to claim 18, wherein the silicone resin is a silicone compound having network structure.

22. A cosmetic material according to claim 21, wherein the silicone compound having network structure is a netted silicone compound containing at least one moiety selected from the group consisting of pyrrolidone, long-chain alkyl, polyoxyalkylene, fluoroalkyl and amino moieties.

23. A skincare cosmetic material comprising the cosmetic material according to any of claims 1 to 22.

24. A hair-care cosmetic material comprising the cosmetic material according to any of claims 1 to 22.

25. An antiperspirant cosmetic material comprising the cosmetic material according to any of claims 1 to 22.

26. A makeup cosmetic material comprising the cosmetic material according to any of claims 1 to 22.

27. An UV protective cosmetic material comprising the cosmetic material according to any of claims 1 to 22.

**28.** A cosmetic material according to any of claims 1 to 27, which is prepared in a state selected from among the states of liquid, emulsion, cream, solid, paste, gel, powder, multilayer, mousse and spray.

## Patentansprüche

**1.** Kosmetisches Material umfassend eine Silikonverbindung, wiedergegeben durch die nachfolgende Formel (1):

$$R^1{}_aR^2{}_bSiO_{(4-a-b)/2} \qquad (1)$$

wobei die $R^1$ Gruppen, die jeweils die gleiche oder verschiedene sein können, jede ein Wasserstoffatom oder eine organische Gruppe darstellen, ausgewählt aus der Klasse bestehend aus Alkylgruppen mit 1 bis 30 Kohlenstoffatomen, Arylgruppen, Aralkylgruppen, fluorinierten Alkylgruppen und organischen Gruppen gemäß der nachfolgenden Formel (2); die $R^2$ Gruppen jeweils eine Silikongruppe wiedergeben gemäß der nachfolgenden Formel (3) und wobei a eine Zahl zwischen 1,0 und 2,5, b eine Zahl zwischen 0,001 und 1,5 ist;

$$-C_cH_{2c}-O-(C_2H_4O)_d(C_3H_6O)_eR^3 \qquad (2)$$

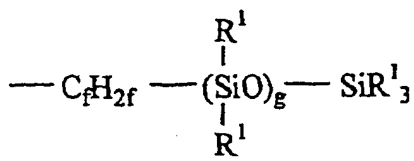

(3)

und wobei $R^3$ eine Kohlenwasserstoffgruppe ist, enthaltend 4 bis 30 Kohlenstoffatome, oder eine organische Gruppe entsprechend $R^4$ - (CO) -; $R^4$ eine Kohlenwasserstoffgruppe ist, enthaltend 1 bis 30 Kohlenstoffatome; c eine ganze Zahl von 0 bis 15, d eine ganze Zahl vom 0 bis 50, e eine ganze Zahl von 0 bis 50, f eine ganze Zahl vom 1 bis 5 und g eine ganze Zahl von 0 bis 500 ist.

**2.** Kosmetisches Material nach Anspruch 1, das darüber hinaus Öle als Bestandteil enthält.

**3.** Kosmetisches Material nach Anspruch 2, wobei wenigstens eines der Öle bei Raumtemperatur flüssig ist.

**4.** Kosmetisches Material nach Anspruch 2, wobei alle oder wenigstens eines der Öle ein Silikonöl ist, enthaltend leicht flüchtige Silikone, ein Silikonöl mit sich wiederholenden Konstitutionsmerkmalen, wiedergegeben durch -$[O-Si]_n$ - in ihrem Molekularskelett oder eine Mischung daraus.

**5.** Kosmetisches Material nach Anspruch 2, 3 oder 4, wobei alle oder wenigstens eines der Öle ein Öl ist, das Fluorin enthaltende Gruppen oder Aminogruppen aufweist.

**6.** Kosmetisches Material nach einem der Ansprüche 1 bis 5, enthaltend zusätzlich wenigstens einen weiteren Bestandteil aus der Gruppe Schaumerzeuger, Pulver und Farbstoffe.

**7.** Kosmetisches Material nach Anspruch 6, bei dem die Schaumerzeuger modifizierte Silikone mit Polyoxyalkylenketten sind.

**8.** Kosmetisches Material nach Anspruch 6 oder 7, wobei die Schaumerzeuger Verbindungen sind, deren HLB Werte im Bereich 2 bis 8 liegen.

**9.** Kosmetisches Material nach einem der Ansprüche 6 bis 8, in dem alle oder ein Teil der Pulver, der Farbstoffe oder der Mischung davon ein organisches Pulver ist, bestehend aus einem Silikonharzpulver, einem Pulver mit einem Silikonelastomer als Gerüst, einem organischen Puder, das sich wiederholende Struktureinheiten enthält, wiedergegeben durch - $[O-Si]_n$ - oder eine Mischung von zwei oder mehreren davon.

10. Kosmetisches Material nach Anspruch 1, **gekennzeichnet dadurch, dass** es darüber hinaus Wasser als Bestandteil enthält.

11. Kosmetisches Material nach einem der Ansprüche 1 bis 10, das zusätzlich als Bestandteil eine Verbindung enthält, die eine alkoholische Hydroxylgruppe in ihrer Molekularstruktur enthält.

12. Kosmetisches Material nach Anspruch 11, bei dem die Verbindung mit einer alkoholischen Hydroxylgruppe in ihrer Molekularstruktur ein monohydratischer wasserlöslicher Alkohol, ein polyhydratischer wasserlöslicher Alkohol oder eine Mischung davon ist.

13. Kosmetisches Material nach Anspruch 11, bei dem die Verbindung mit einer alkoholischen Hydroxylgruppe in ihrer Molekularstruktur ein wasserlösliches Polymer ist.

14. Kosmetisches Material nach einem der Ansprüche 1 bis 13, das zusätzlich als Bestandteile quervernetzte Organopolysiloxane enthält.

15. Kosmetisches Material nach Anspruch 14, bei dem die quervernetzten Organopolysiloxane quervernetzte Organopolysilixane sind, die ein Aufquellen verursachen, wenn sie ein Silikon mit geringer Viskosität zwischen 0,65 und 10,0 $mm^2$/sek (25°) in einer Menge größer als ihr Eigengewicht enthalten.

16. Kosmetisches Material nach Anspruch 14 oder 15, wobei die quervernetzten Organopolysiloxane Organopolysiloxane sind, die eine vernetzte Struktur aufweisen, gebildet durch die Reaktion zwischen den direkt an die Siliziumatome gebundenen Wasserstoffatomen und einem Vernetzungsmittel das wenigstens zwei vinylische reaktive Anteile pro Molekül enthält.

17. Kosmetisches Material nach einem der Ansprüche 14 bis 16, bei dem die vernetzten Organopolysiloxane solche sind, die in ihren Vernetzungen wenigstens eine Art von Anteilen aufweisen, ausgewählt aus der Familie bestehend aus Polyoxyalkylen, Alkyl, Alkenyl, Aryl und Fluoralkylanteilen.

18. Kosmetisches Material nach einem der Ansprüche 1 bis 17, das zusätzlich als Bestandteil Silikonharz enthält.

19. Kosmetisches Material nach Anspruch 18, bei dem das Silikonharz ein Arylsilikonharz ist.

20. Kosmetisches Material nach Anspruch 18 oder 19, wobei das Silikonharz ein Arylsilikonharz ist, enthaltend wenigstens einen Anteil ausgewählt aus der Gruppe bestehend aus Pyrrolidon, Langkettenalkyl, Polyoxyalkylen und Fluoroalkylanteilen.

21. Kosmetisches Material nach Anspruch 18, wobei das Silikonharz eine Silikonverbindung mit einer Netzwerkstruktur ist.

22. Kosmetisches Material nach Anspruch 21, wobei die Silikonverbindung mit Netzwerkstruktur eine netzförmige Silikonverbindung ist, die wenigstens einen Anteil aus der Gruppe enthält, bestehend aus Pyrrolidon, Langkettenalkyl, Polyoxyalkylen, Fluoroalkyl und Aminoanteilen.

23. Kosmetisches Hautpflegemittel, umfassend ein Kosmetisches Mittel nach einem der Ansprüche 1 bis 22.

24. Kosmetisches Haarpflegemittel, umfassend das kosmetische Mittel nach einem der Ansprüche 1 bis 22.

25. Kosmetisches Antischweißmittel, umfassend das kosmetische Material nach einem der Ansprüche 1 bis 22.

26. Kosmetisches Make-up Mittel, umfassend das Kosmetische Material nach einem der Ansprüche 1 bis 22.

27. UV-schützendes kosmetisches Mittel, umfassend das kosmetische Material nach einem der Ansprüche 1 bis 22.

28. Kosmetisches Mittel nach einem der Ansprüche 1 bis 27, zubereitet in einem Zustand der ausgewählt ist aus den Zuständen Flüssigkeit, Emulsion, Creme, Feststoff, Paste, Gel, Puder, Multischicht, Mousse und Spray.

## Revendications

**1.** Matériau cosmétique contenant un composé silicone représenté par la formule (1) suivante :

$$R^1_a R^2_b SiO_{(4-a-b)/2} \quad (1)$$

dans laquelle les groupes $R^1$, qui sont identiques ou différents, représentent chacun un atome d'hydrogène ou un groupe organique choisi parmi la classe comprenant les groupes alkyle contenant de 1 à 30 atomes de carbone, les groupes aryle, les groupes aralkyle, les groupes alkyle fluorés et les groupes organiques représentés par la formule (2) suivante ; les groupes $R^2$ représentent chacun un groupe silicone représenté par la formule (3) suivante ; a est un nombre de 1,0 à 2,5 ; b est un nombre de 0,001 à 1,5 ;

$$-C_cH_{2c}-O-(C_2H_4O)_d(C_3H_6O)_eR^3 \quad (2)$$

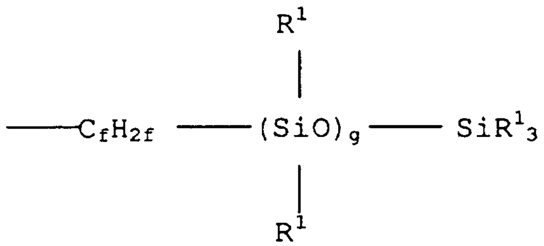

(3)

dans lequel $R^3$ est un groupe hydrocarboné contenant de 4 à 30 atomes de carbone, ou un groupe organique représenté par $R^4$-(CO)- ; $R^4$ est un groupe hydrocarboné contenant de 1 à 30 atomes de carbone ; c est un nombre entier de 0 à 15, d est un nombre entier de 0 à 50 et e est un nombre entier de 0 à 50 ; et f est un nombre entier de 1 à 5, et g est un nombre entier de 0 à 500.

**2.** Matériau cosmétique selon la revendication 1, contenant en outre des huiles en tant que constituant.

**3.** Matériau cosmétique selon la revendication 2, dans lequel au moins une des huiles est à l'état liquide à température ambiante.

**4.** Matériau cosmétique selon la revendication 2, dans lequel toutes les huiles sont, ou au moins une des huiles est, une huile de silicone contenant des silicones volatiles, une huile de silicone ayant des motifs répétitifs constitutionnels représentés par $-[O-Si]_n-$ dans son squelette moléculaire, ou un mélange de ces dernières.

**5.** Matériau cosmétique selon la revendication 2, 3 ou 4, dans lequel toutes les huiles sont, ou au moins une des huiles est, une huile des groupes contenant du fluor ou des groupes amino.

**6.** Matériau cosmétique selon l'une quelconque des revendications 1 à 5, contenant en outre au moins un ingrédient choisi dans le groupe contenant des agents tensioactifs, des poudres et des colorants.

**7.** Matériau cosmétique selon la revendication 6, dans lequel les agents tensioactifs sont des silicones modifiées ayant des chaînes polyoxyalkylène.

**8.** Matériau cosmétique selon la revendication 6 ou 7, dans lequel les agents tensioactifs sont des composés dont les valeurs HLB se situent dans l'intervalle de 2 à 8.

**9.** Matériau cosmétique selon l'une quelconque des revendications 6 à 8, dans lequel tout ou partie de la poudre, du colorant ou du mélange de ces derniers est une poudre organique constituée d'une poudre de résine silicone, une poudre ayant un élastomère de silicone en tant que squelette, une poudre organique contenant des unités répétitives constitutionnelles représentées par $-[O-Si]_n-$ ou un mélange de deux ou plusieurs de ces dernières.

**10.** Matériau cosmétique selon la revendication 1, contenant en outre de l'eau comme constituant.

**11.** Matériau cosmétique selon l'une quelconque des revendications 1 à 10, contenant en outre en tant que constituant un composé ayant un groupe hydroxyle alcoolique dans sa structure moléculaire.

**12.** Matériau cosmétique selon la revendication 11, dans lequel le composé ayant un groupe hydroxyle alcoolique dans sa structure moléculaire est un monoalcool hydrosoluble, un polyol hydrosoluble ou un mélange de ces derniers.

**13.** Matériau cosmétique selon la revendication 11, dans lequel le composé ayant un groupe hydroxyle . alcoolique dans sa structure moléculaire est un polymère hydrosoluble.

**14.** Matériau cosmétique selon l'une quelconque des revendications 1 à 13, contenant en outre des organopolysiloxanes réticulés en tant que constituant.

**15.** Matériau cosmétique selon la revendication 14, dans lequel les organopolysiloxanes réticulés sont des organopolysiloxanes réticulés qui provoquent un gonflement lorsqu'ils contiennent une silicone ayant une faible viscosité de 0,65 à 10,0 $mm^2/s$ (25°) en une quantité supérieure à leur propre poids.

**16.** Matériau cosmétique selon la revendication 14 ou 15, dans lequel les organopolysiloxanes réticulés sont des organopolysiloxanes réticulés ayant une structure réticulée formée par la réaction entre les atomes d'hydrogène directement liés aux atomes de silicium et un agent de réticulation ayant au moins deux fragments réactifs vinyliques par molécule.

**17.** Matériau cosmétique selon l'une quelconque des revendications 14 à 16, dans lequel les organopolysiloxanes réticulés sont des organopolysiloxanes réticulés ayant dans leurs liaisons réticulaires au moins une sorte de fragment choisi parmi la famille comprenant des fragments de polyoxyalkylène, d'alkyle, d'alcényle, d'aryle et de fluoroalkyle.

**18.** Matériau cosmétique selon l'une quelconque des revendications 1 à 17, contenant en outre de la résine silicone en tant que constituant.

**19.** Matériau cosmétique selon la revendication 18, dans lequel la résine silicone est une résine silicone/acrylique

**20.** Matériau cosmétique selon la revendication 18 ou 19, dans lequel la résine silicone est une résine silicone/acrylique contenant au moins un fragment choisi dans le groupe comprenant des fragments de pyrrolidone, d'alkyle à chaîne longue, de polyoxyalkylène et de fluoroalkyle.

**21.** Matériau cosmétique selon la revendication 18, dans lequel la résine silicone est un composé silicone ayant une structure réticulée.

**22.** Matériau cosmétique selon la revendication 21, dans lequel le composé silicone ayant une structure réticulée est une silicone réticulée contenant au moins un fragment choisi dans le groupe contenant des fragments de pyrrolidone, d'alkyle à chaîne longue, de polyoxyalkylène, de fluoroalkyle et d'amine.

**23.** Matériau cosmétique de soins pour la peau contenant le matériau cosmétique selon l'une quelconque des revendications 1 à 22.

**24.** Matériau cosmétique de soins pour les cheveux contenant le matériau cosmétique selon l'une quelconque des revendications 1 à 22.

**25.** Matériau cosmétique anti-transpirant contenant le matériau cosmétique selon l'une quelconque des revendications 1 à 22.

**26.** Matériau cosmétique de maquillage contenant le matériau cosmétique selon l'une quelconque des revendications 1 à 22.

**27.** Matériau cosmétique protecteur contre les UV contenant le matériau cosmétique selon l'une quelconque des revendications 1 à 22.

**28.** Matériau cosmétique selon l'une quelconque des revendications 1 à 27, qui est préparé dans un état choisi parmi les états suivants : liquide, émulsion, crème, solide, pâte, gel, poudre, multicouche, mousse et spray.